# FASCICULE DE BREVET EUROPEEN

(11) **EP 0 842 184 B1**
(45) Date de publication et mention de la délivrance du brevet: **29.01.2003**
(21) Numéro de dépôt: 97915528.0
(22) Date de dépôt: 20.03.1997
(51) Int. Cl.: C07H 1/08, C12Q 1/68, G01N 27/447

(54) **ISOLEMENT DE L'ACIDE NUCLEIQUE**
ISOLIERUNG VON NUKLEINSÄURE
NUCLEIC ACID ISOLATION

(30) Priorité: 20.03.1996 FR 9603753; 09.04.1996 FR 9604691
(43) Date de publication de la demande: 20.05.1998
(73) Titulaire: BIO MERIEUX, 69280 Marcy l'Etoile (FR)
(72) Inventeur: CROS, Philippe, F-69005 Lyon (FR); ELAISSARI, Abdelhamid, F-69007 Lyon (FR); MABILAT, Claude, F-69140 Rillieux-la-Pape (FR); PICHOT, Christian, F-69960 Corbas (FR); RODRIGUE, Marc, F-69570 Dardilly (FR); SANTORO, Lise, F-69110 Sainte-Foy-Les-Lyons (FR)
(74) Mandataire: Guerre, Dominique
(86) Numéro de dépôt international: FR9700496
(87) Numéro de publication internationale: WO97034909

(56) Documents cités:
- EP-A- 0 161 881
- EP-A- 0 366 241
- EP-A- 0 501 301
- DE-A- 3 717 209
- US-A- 4 767 700
- US-A- 4 997 932
- POLYMERS FOR ADVANCED TECHNOLOGIES, vol. 6, no. 7, 1995, CHICHESTER GB, pages 489-496, XP000518430 F. MEUNIER ET AL.: "Preparation and characterization of cationic poly(N-isopropylacrylamide) copolymer latexes" cité dans la demande

## Description

La présente invention appartient au domaine de la purification des acides nucléiques, en milieu aqueux.

On connaît selon le document WO-A-95/04140 un procédé pour purifier, en milieu aqueux, des acides nucléiques présents dans un échantillon, selon lequel on met en contact ledit échantillon avec un système particulaire consistant en des billes de silice, en présence d'une substance chaotropique, puis on sépare de la solution aqueuse finale les acides nucléiques fixés sur les billes.

Conformément au document F. MEUNIER et al., Polymers for Advanced Technologies, Vol 6, pp 489-496, (1995), on décrit la préparation d'un polymère dénommé PNIPAM, par polymérisation de (1) N-isopropylacrylamide, (2) N,N-méthylène bisacrylamide et (3) chlorure de 2-aminoéthyl-méthacrylate, en présence d'un amorceur de polymérisation. Le comportement de ce polymère fonctionnalisé en surface peut le rendre particulièrement adapté à une fixation par covalence, de molécules biologiques.

Le document EP-A-0 161 881 enseigne qu'un polymère thermosensible tel que les polymères obtenus par copolymérisation de monomères de N-alkyl- ou de N-alkylène-acrylamide ou méthacrylamide et de monomères de dérivés acryliques ou méthacrylique, peut être utilisé dans l'isolement de matériel biologique, grâce à sa capacité à changer de structure en fonction de la température. Il présente une structure déployée à basse température, qui facilite la fixation d'un matériel biologique, et une structure rétractée à haute température, qui permet la libération du matériel biologique fixé. Le contrôle des étapes de fixation et de libération du matériel biologique peut donc être effectué par variation de la température. Pour un meilleur contrôle, on peut en outre faire varier le pH.

L'utilisation proposée par ce document s'étend à l'isolement de tout matériel biologique présent dans un échantillon, et notamment matériel nucléique et matériel protéique, sans aucune spécificité.

Selon l'invention, on apporte un procédé d'isolement sélectif d'un matériel nucléique, présent dans un échantillon. Même si l'échantillon est complexe et contient un matériel protéique et/ou des inhibiteurs de réaction enzymatique, le procédé de l'invention limite voire supprime tout isolement du matériel protéique et/ou desdits inhibiteurs, tout en favorisant l'isolement du matériel nucléique.

Un procédé d'isolement en phase aqueuse, selon l'invention, d'un matériel nucléique présent dans un échantillon, comprend les étapes suivantes :
selon une étape (a) dite d'obtention du réactif d'adsorption, on dispose d'un réactif d'adsorption comprenant un sol constitué par une phase continue aqueuse et une phase discontinue du support particulaire qui comprend un polymère particulaire, fonctionnalisé, ledit polymère étant obtenu par polymérisation de (1) un premier monomère hydrosoluble, d'acrylamide ou d'un dérivé d'acrylamide, (2) au moins un *agent* de réticulation et (3) au moins un second monomère, fonctionnel, cationique et hydrosoluble, ledit polymère présentant une température critique inférieure de solubilité (LCST) prédéterminée qui est comprise entre 25 et 45°C, de préférence entre 30 et 40°C,
selon une étape (b) dite de mise en contact, on met en contact le réactif d'adsorption avec l'échantillon contenant le matériel nucléique,
selon une étape (c) dite d'adsorption, pour la mise en contact selon (b), on choisit au moins un et de préférence au moins deux des paramètres suivants pour le milieu réactionnel:
   - pH au plus égal à 7,
   - force ionique au plus égale à 10⁻² M,
   - température inférieure à la LCST du polymère,
selon une étape (d) dite de séparation, après éventuellement avoir observé que l'adsorption a eu lieu, on sépare, de la phase continue, la phase discontinue et notamment celle ayant adsorbé le matériel nucléique,
selon une étape (e) dite de désorption, on dissocie, par désorption, le matériel nucléique par rapport au support particulaire, en augmentant la force ionique jusqu'à une force ionique supérieure à 10⁻²M.

Avantageusement, pour l'étape (e) de désorption, on fait en outre varier au moins un des paramètres choisis parmi le pH et la température comme suit :
- augmentation du pH jusqu'à un pH supérieur à 7,
- augmentation de la température jusqu'à une température supérieure à la LCST du polymère.

L'invention concerne aussi un procédé d'isolement, en phase aqueuse, d'un matériel nucléique, présent dans un échantillon, comprenant une étape d'adsorption dudit matériel nucléique, sur un support particulaire, permettant une utilisation en l'état du matériel nucléique adsorbé sur le support particulaire, dans une étape ultérieure d'analyse. Ce procédé comprend les étapes suivantes :
selon une étape (a) dite d'obtention du réactif d'adsorption, on dispose d'un réactif d'adsorption comprenant un sol constitué par une phase continue aqueuse et une phase discontinue du support particulaire qui comprend un polymère particulaire, fonctionnalisé, ledit polymère étant obtenu par polymérisation de (1) un premier monomère hydrosoluble, d'acrylamide ou d'un dérivé d'acrylamide, (2) au moins un agent de réticulation et (3) au moins un second monomère, fonctionnel, cationique et hydrosoluble, et ledit polymère présentant une température critique inférieure de solubilité (LCST) prédéterminée qui est comprise entre 25 et 45°C,
selon une étape (b) dite de mise en contact, on met en contact le réactif d'adsorption avec l'échantillon contenant le matériel nucléique,
selon une étape (c) dite d'adsorption, on choisit, pour la mise en contact selon (b), une force ionique au plus égale à 10⁻² M pour le milieu réactionnel,
selon une étape (d) dite de séparation, après éventuellement avoir observé que l'adsorption a eu lieu, on sépare la phase discontinue de la phase continue, procédé selon lequel l'étape de désorption est facultative.

Conformément à une mise en oeuvre préférentielle ce dernier procédé, selon l'étape (c) d'adsorption, on choisit en outre, pour la mise en contact selon (b), au moins un des paramètres suivants pour le milieu réactionnel :
- pH au plus égal à 7,
- température inférieure à la LCST du polymère.

Bien entendu, ce procédé peut comprendre, après l'étape (d) de séparation, une étape dite de désorption, selon laquelle on dissocie, par désorption, le matériel nucléique par rapport au support particulaire, en faisant varier au moins un des paramètres choisis parmi la force ionique, le pH et la température, comme suit :
- augmentation de la force ionique jusqu'à une force ionique supérieure à 10⁻²M,
- augmentation du pH jusqu'à un pH supérieur à 7,
- augmentation de la température jusqu'à une température supérieure à la LCST du polymère.

On fait avantageusement varier au moins la force ionique.

Les procédés définis ci-dessus selon l'invention seront préférentiellement mis en oeuvre selon deux variantes relatives à l'étape (a).

Selon une première variante qui sera illustrée dans les Exemples, le support particulaire consiste en ledit polymère particulaire, et dans ce cas le ou les agents de réticulation (2) sont hydrosolubles.

Selon une seconde variante, le support particulaire comprend en outre un noyau organique ou inorganique, recouvert en totalité ou en partie par ledit polymère particulaire, ledit noyau ne modifiant pas les propriétés d'adsorption du polymère vis-à-vis dudit matériel nucléique. Le noyau ou partie du noyau remplit alors la fonction de l'agent de réticulation (2), un autre agent de réticulation du type agent de réticulation hydrosoluble pouvant être prévu. A titre d'exemple, le noyau peut être un noyau de polystyrène, et/ou comprendre un composé magnétique.

Selon une mise en oeuvre particulière et préférentielle de ces procédés, on ajoute dans l'échantillon avant l'étape (b), ou dans le milieu réactionnel après l'étape (b), et notamment après l'étape (c) ou l'étape (d), au moins une sonde et/ou une amorce susceptible de s'hybrider spécifiquement sur le matériel nucléique avant ou après l'étape (b).

Dans une autre mise en oeuvre particulière, le matériel nucléique consiste en une sonde ou une amorce, et selon (b) et (c) on met en contact le réactif d'adsorption avec ledit matériel nucléique, pour obtenir un réactif d'hybridation, puis selon (b') après éventuellement avoir observé que l'adsorption a eu lieu, et séparé du milieu réactionnel le réactif d'hybridation, on met en contact ledit réactif d'hybridation avec un milieu contenant au moins un acide nucléique ou fragment d'acide nucléique, dans des conditions adaptées pour l'hybridation ou l'élongation de l'amorce.

Le polymère particulaire est avantageusement obtenu par polymérisation radicalaire, en présence d'un amorceur de polymérisation, cationique ou neutre, et hydrosoluble.

Le premier monomère (1) est de préférence choisi parmi les N-alkylacrylamides et les N,N-dialkylacrylamides, et plus particulièrement parmi le N-isopropylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacrylamide, le premier monomère étant de préférence le N-isopropylacrylamide (NIPAM).

Le ou les seconds monomères, fonctionnels (3) sont de préférence choisis parmi les dérivés acryliques et méthacryliques, le chlorure de 2-aminoéthyl méthacrylate (AEM), les dérivés de N-vinyl-pyridine, les dérivés de trialkylammonium et les dérivés de chlorure d'isothiouronium.

Avantageusement l'agent de réticulation hydrosoluble (2) est choisi parmi le N,N-méthylène bisacrylamide (MBA), l'éthylène glycol diméthacrylate, et l'amorceur de polymérisation est le chlorure de 2,2'-azobis amidino-propane (V50).

L'étape (d) de séparation est de préférence effectuée selon une technique choisie parmi la centrifugation, la filtration, la précipitation, la sédimentation et l'application d'un champ magnétique.

Avant l'étape (d) de séparation on peut éventuellement observer que la réaction d'adsorption s'est produite. A titre d'exemple, on peut utiliser les techniques de HPLC ou d'électrophorèse capillaire.

Avant d'exposer plus en détail l'invention, certains termes employés dans la présente description et dans les revendications sont ci-après définis :

Par isolement d'un matériel nucléique selon l'invention, on comprend la séparation, la détection de ce matériel, l'enrichissement d'une fraction en matériel nucléique, selon une méthode d'isolement spécifique ou aspécifique, de manière qualitative et/ou quantitative.

Un matériel nucléique selon l'invention est un acide nucléique, un fragment d'acide nucléique, un mélange d'acides nucléiques et/ou de fragments d'acides nucléiques, ou une fraction d'acides nucléiques et/ou de fragments d'acides nucléiques. Par acide nucléique, on entend tout acide nucléique, sous forme libre ou combinée éventuellement à des protéines, quelle que soit son origine cellulaire, bactérienne, virale ou autre. Il s'agit indifféremment d'un acide désoxyribonucléique ou d'un acide ribonucléique, constitué par un enchaînement de nucléotides naturels dont les éléments constitutifs sont un sucre, un groupement phosphate et une base azotée choisie parmi l'adénine, la guanine, l'uracile, la cytosine, la thymine, et/ou de nucléotides modifiés dans l'un au moins des trois éléments constitutifs ; à titre d'exemple, la modification peut intervenir au niveau des bases, générant des bases modifiées, telles que l'inosine, la méthyl-5-désoxycytidine, la désoxyuridine, la diméthylamino-5-désoxyuridine, la diamino-2,6-purine, la bromo-5-désoxyuridine, et telles que des bases modifiées par un traceur détectable directement ou indirectement par des techniques connues de l'homme du métier, à titre d'exemple les bases modifiées par la biotine ; au niveau du sucre, à savoir le remplacement d'au moins un désoxyribose par un polyamide ; et/ou au niveau du groupement phosphate par exemple son remplacement par des esters notamment choisis parmi les esters de diphosphate, d'alkyl- et aryl-phosphonate et d'alkyl- et aryle-phosphorothioate. L'acide nucléique selon l'invention est totalement ou partiellement monocaténaire et/ou bicaténaire, en particulier il peut consister en un duplex sonde-acide nucléique, sonde-fragment d'acide nucléique, amorce-acide nucléique ou amorce-fragment d'acide nucléique ; le duplex peut être un homoduplex ou un hétéroduplex.

L'invention est bien sûr appliquée à l'isolement de fragments d'acides nucléiques tels que définis ci-dessus, ou oligonucléotides (ODN), de tailles variables.

Le matériel nucléique peut être d'origine 5 naturelle, et/ou obtenu par recombinaison génétique et/ou par synthèse chimique, à titre d'exemple il peut consister en une sonde ou une amorce.

La présente invention est appliquée à l'isolement aspécifique d'une fraction d'acides nucléiques et/ou de fragments d'acides nucléiques, contenue dans un échantillon, mais aussi à l'isolement spécifique d'un acide nucléique ou d'un fragment d'acide nucléique, ou d'un mélange d'acides nucléiques ou de fragments d'acides nucléiques, présents dans un échantillon.

Un échantillon tel qu'on l'entend selon l'invention, comprend tout échantillon susceptible de contenir un matériel nucléique, notamment un échantillon biologique tel que celui obtenu à partir d'un fluide biologique, un échantillon d'origine alimentaire. L'échantillon consiste en tout ou partie d'un échantillon, en particulier il peut consister en un aliquote, une dilution. L'échantillon peut ou non avoir été soumis à un traitement préalable notamment de purification ou de lyse afin de faciliter la libération des acides nucléiques.

La LCST d'un polymère tel que celui qui fait l'objet de la présente invention est notamment définie et mesurée par des techniques décrites dans les documents suivants : Hiroshi Inomata et al., Macromolecules 1994, 27, 6459-6464.

Une sonde est un fragment nucléotidique possédant une spécificité d'hybridation dans des conditions déterminées pour former un complexe d'hybridation avec un fragment nucléotidique. Une sonde utilisée dans le cadre de la présente invention sera de préférence une sonde de capture, sans pour autant exclure de ce cadre les autres types de sondes.

Par amorce selon l'invention, on entend une sonde possédant une spécificité d'hybridation dans des conditions déterminées pour l'initiation d'une polymérisation enzymatique par exemple dans une technique d'amplification telle que la PCR (Polymerase Chain Reaction), la technique dite NASBA ("Nucleic Acid Sequence-Based Amplification) ou encore la technique dite TMA (Transcription Mediated Amplification), dans un procédé d'élongation, tel que le séquençage, dans une méthode de transcription inverse ou analogue.

Par dérivé acrylamide selon l'invention, on entend un monomère polymérisable répondant à la formule R⁰-CH=C(R¹)-CONR²R³, dans laquelle R⁰, R¹, R² et R³ représentent un groupe indépendamment choisi parmi l'hydrogène, les groupes hydrocarbonés inférieurs, linéaires ou ramifiés, aliphatiques ou cycliques, les groupes hétérocycliques azotés tels que l'imidazole.

L'adsorption de matériel nucléique telle qu'entendue selon la présente invention est définie comme suit : un matériel nucléique est adsorbé sur un support particulaire si après un temps de contact entre ledit matériel et ledit support, au moins un des groupes appartenant aux éléments constitutifs du matériel nucléique est fixé à la surface du support ; l'adsorption résulte d'interactions ioniques et/ou de liaisons hydrogène, et éventuellement d'interactions hydrophobes, à l'exclusion de toute liaison covalente, entre le matériel et le support.

Enfin par polymère fonctionnalisé, on entend un polymère présentant au moins une interface portant des groupes fonctionnels susceptibles de générer avec des groupes des éléments constitutifs du matériel nucléique, l'une quelconque des interactions et/ou liaisons impliquées dans le phénomène d'adsorption. De préférence ces groupes fonctionnels sont choisis parmi NH₃⁺ ; NH₄⁺ ; NR₃⁺ où R représente un groupe hydrocarboné, saturé ou insaturé, aliphatique ou cyclique, NR₃⁺ pouvant représenter le groupe pyridinium ; et le groupe isothiouronium.

La présente invention est à présent décrite en référence aux Exemples 1 à 6 et aux Figures 1 à 7 présentées ci-après :
Figure 1 représente la variation de l'interface du polymère en fonction du pH et de la température,
Figure 2 représente l'effet du pH et de la température sur l'adsorption de l'ARN,
Figure 3 représente l'effet du pH à 40°C sur l'adsorption de la BSA,
Figure 4 représente l'effet de la force ionique et de la température sur l'adsorption de l'ARN,
Figure 5 représente l'effet du pH à 20°C sur la désorption de l'ARN,
Figure 6 représente l'effet du pH à 40°C sur la désorption de l'ARN, et
Figure 7 représente l'effet de la force ionique à pH 9,2 et à 20°C sur la désorption de l'ARN.

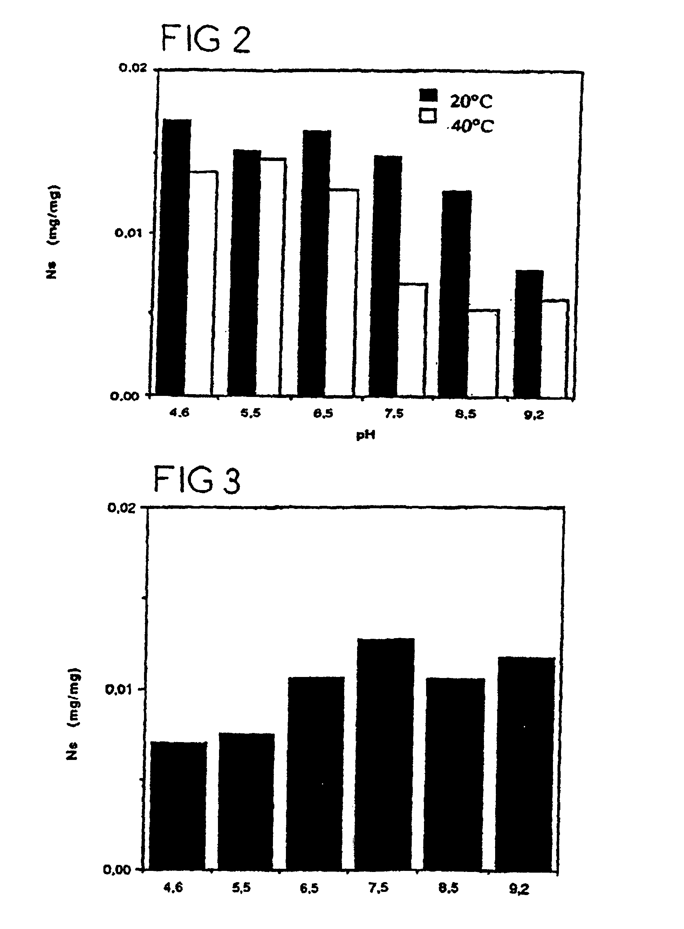

Pour les figures 2 à 4, la valeur Ns correspond à la quantité de l'entité biologique fixée sur le polymère et est exprimée en milligrammes de molécules biologiques fixées par milligramme de polymère.

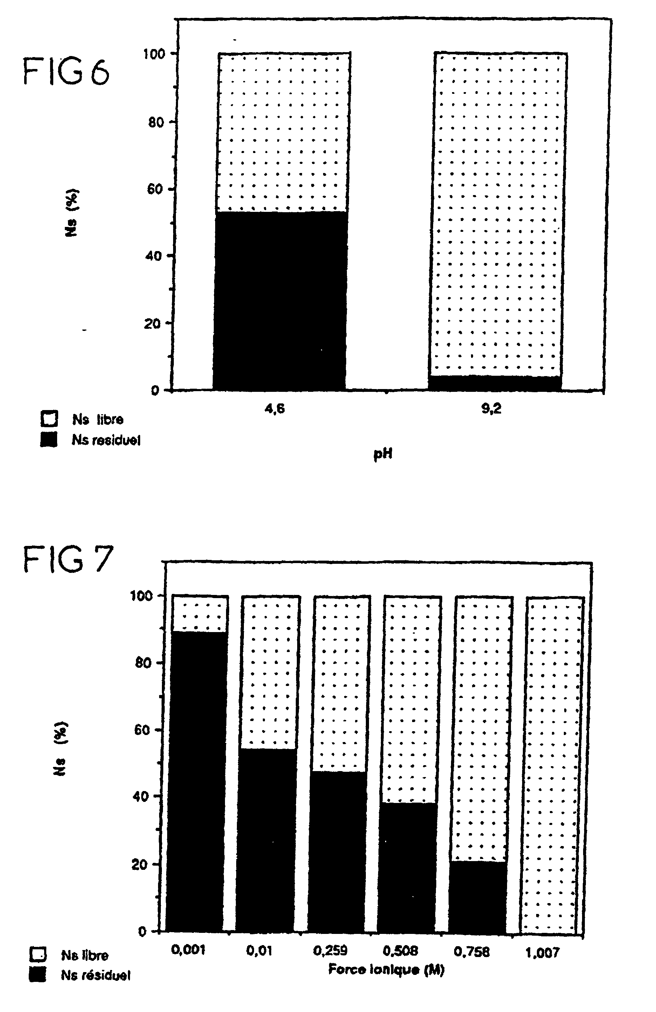

Pour les figures 5 à 7, la valeur Ns correspond au pourcentage d'ARN libérés (Ns libre) ou d'ARN non libérés (Ns résiduel), par rapport à la quantité d'ARN préalablement adsorbée sur les particules conformément à l'exemple 2.

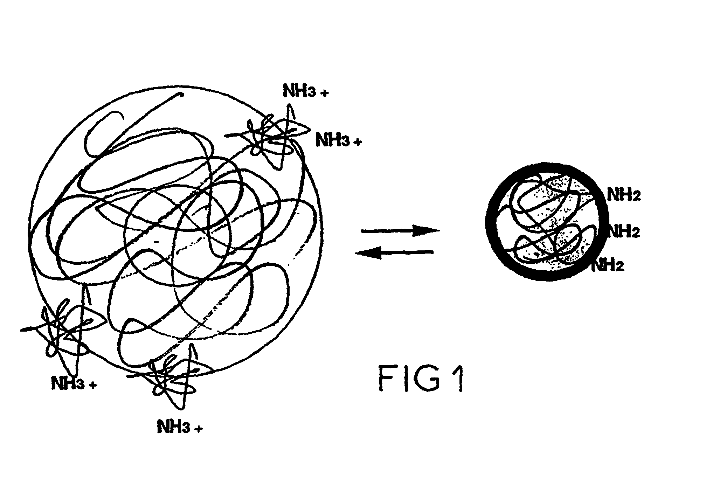

Comme les exemples suivants l'illustreront, les conditions de pH, de force ionique et/ou de température au cours de l'étape (c) d'adsorption sont déterminantes. En effet, comme on peut l'observer sur la Figure 1, endessous d'une valeur de pH égale à 7 et de température inférieure à la LCST du polymère, le polymère présente une chevelure hydrophile, chargée, alors qu'au-dessus d'une valeur de pH égale à 7 et de température supérieure à la LCST le polymère présente une conformation rétractée hydrophobe et neutre, ce qui entraîne une diminution de l'adsorption des acides nucléiques et à la fois une adsorption croissante des protéines.

### EXEMPLE 1 : PRÉPARATION D'UN POLYMÈRE À BASE DE NIPAM

Trois techniques de polymérisation ont été utilisées pour la préparation de ce polymère :
1) polymérisation en batch (ou procédé en réacteur fermé);
2) polymérisation en semi-continu et 3) polymérisation sur semence. Dans chacune de ces techniques, les mêmes réactifs suivants ont été utilisés :
   * Premier monomère : N-isopropylacrylamide (NIPAM) commercialisé par Kodak,
   * Réticulant : N,N-méthylène bisacrylamide (MBA) disponible chez Aldrich,
   * Amorceur : chlorure de 2,2'-azobis amidino propane (V50) commercialisé par Wako,
   * Sel pour ajuster la force ionique : NaCl (Prolabo),
   * Second monomère, fonctionnel : chlorure de 2-aminoéthyl méthacrylate (AEM) commercialisé par Kodak.

### 1) Polymérisation en batch

Le premier monomère (NIPAM), le second monomère, fonctionnel (AEM) et le réticulant (MBA) sont introduits ensemble en une seule étape avant que la polymérisation ne soit amorcée par addition de l'amorceur (V50) qui se décompose sous l'effet de la chaleur en produisant des radicaux libres. La durée de polymérisation est de 30 min.

La formulation du polymère obtenu à qui on a affecté la référence PNIPAM42 est la suivante :

| | |
|---|---|
| volume total^{(a)} | 250 ml |
| NIPAM | 48,51 mmoles |
| MBA | 3 mmoles |
| AEM | 0,48 mmoles |
| V50 | 0,30 mmoles |
| Température | 70°C |

| | |
|---|---|
| (a) eau bouillie et dégazée | |

Les caractéristiques du polymère obtenu sont reportées dans le tableau I suivant:

**Tableau I**

| diamètre ^{(a)} DDL 20°C | diamètre ^{(b)} taille DDL 40°C | diamètre ^{(c)} MET | concentration en AEM ^{(d)} | LCST ^{(e)} | CCC ^{(f)} à 20°C |
|---|---|---|---|---|---|
| 292 nm | 164 nm | 129 nm | 14,1 µmol/g de polymère | 31,5°C | 1.00 mole/l |

| | | | | | |
|---|---|---|---|---|---|
| (a) diamètre mesuré par diffusion dynamique de la lumière à 20°C | | | | | |
| (b) diamètre mesuré par diffusion dynamique de la lumière à 40°C | | | | | |
| (c) diamètre mesuré par microscopie électronique à transmission | | | | | |
| (d) densité de charge exprimée en µmole (amine primaire)/g de polymère | | | | | |
| (e) température critique inférieure de solubilité (LCST) déterminée par mesure de turbidité en fonction de la température | | | | | |
| (f) concentration critique de coagulation (CCC) à 20°C déterminée par mesure de turbidité en fonction de la salinité (NaCl). | | | | | |

### 2) Polymérisation en semi-continu

Une partie de second monomère, fonctionnel est introduite dans le réacteur sur une période comprise entre le début de la polymérisation et la fin de la conversion totale de celle-ci. Cet ajout peut être effectué à une vitesse d'injection constante (polymérisation par ajout continu) ou bien suivant un ajout bien contrôlé à des intervalles réguliers (polymérisation en semi-continu). Le but de cette méthode de polymérisation est d'augmenter l'incorporation de second monomère, fonctionnel (chargé) sans augmenter le pourcentage de polymère hydrosoluble dans le milieu réactionnel qui pourrait perturber le déroulement de la polymérisation.

La formulation du polymère obtenu à qui on a affecté la référence PNIPAM45 est la suivante :

| | |
|---|---|
| volume total^{(a)} | 250 ml |
| NIPAM | 48,51 mmoles |
| MBA | 3 mmoles |
| AEM | 0,48 mmoles |
| V50 | 0,30 mmoles |
| Température | 70°C |
| ajouts | entre 3 et 6 min |

| | |
|---|---|
| (a) eau bouillie et dégazée | |

Les caractéristique du polymère PNIPAM45 obtenu sont reportées dans le tableau II suivant :

**Tableau II**

| diamètre ^{(a)} DDL 20°C | diamètre ^{(b)} taille DDL 40°C | diamètre ^{(c)} MET | concentration en AEM^{(d)} | LCST^{(e)} | CCC ^{(f)} à 20°C |
|---|---|---|---|---|---|
| 823 nm | 530 nm | 327 nm | 10.0 µmol/g de polymère | 32 °C | 1.00 mole/l |

| | | | | | |
|---|---|---|---|---|---|
| (a) diamètre mesuré par diffusion dynamique de la lumière à 20°C | | | | | |
| (b) diamètre mesuré par diffusion dynamique de la lumière à 40°C | | | | | |
| (c) diamètre mesuré par microscopie électronique à transmission | | | | | |
| (d) densité de charge exprimée en µmole (amine primaire)/g de polymère | | | | | |
| (e) température critique inférieure de solubilité (LCST) déterminée par mesure de turbidité en fonction de la température | | | | | |
| (f) concentration critique de coagulation (CCC) à 20°C déterminée par mesure de turbidité en fonction de la salinité (NaCl). | | | | | |

### 3) Polymérisation sur semence

Cette technique consiste à introduire le second monomère, fonctionnel dans un milieu réactionnel contenant un polymère préalablement préparé et parfaitement caractérisé. Le second monomère, fonctionnel peut être additionné seul ou en mélange avec le ou les monomère(s) ou les comonomères, en une étape ou en semi-continu.

La formulation du polymère obtenu à qui on a affecté la référence PNIPAM94 est la suivante :

Un volume de 40 ml de semence à un taux de solide de 4,5 % est utilisé. Les réactifs ont été ajoutés dilués dans un volume de 5 ml d'eau. Les pourcentage molaires de NIPAM, de MBA et de V50 ajoutés dans la deuxième étape sont identiques à ceux de la semence (cf 1)). En revanche, la concentration en second monomère, fonctionnel est contrôlée (augmentée ou diminuée suivant la densité de charge voulue) ; dans ce cas 10 % (mole) de AEM sont ajoutés par rapport au premier monomère NIPAM.

Les caractéristiques du polymère PNIPAM94, obtenu après réensemencement à partir de la semence inscrite sous la référence PNIPAM93 synthétisée suivant le mode opératoire décrit dans 1), sont reportées dans le tableau III suivant :

**Tableau III**

| diamètre ^{(a)} DDL 20°C | diamètre ^{(b)} taille DDL 40°C | diamètre ^{(c)} MET | concentration en AEM ^{(d)} | LCST^{(e)} | CCC ^{(f)} à 20°C |
|---|---|---|---|---|---|
| 504 nm | 290 nm | 176 nm | 22.4 µmol/g de polymère | 32°C | 1.10 mole/l |

| | | | | | |
|---|---|---|---|---|---|
| (a) diamètre mesuré par diffusion dynamique de la lumière à 20°C | | | | | |
| (b) diamètre mesuré par diffusion dynamique de la lumière à 40°C | | | | | |
| (c) diamètre mesuré par microscopie électronique à transmission | | | | | |
| (d) densité de charge exprimée en µmole(amine primaire)/g de polymère | | | | | |
| (e) température critique inférieure de solubilité (LCST) déterminée par mesure de turbidité en fonction de la température | | | | | |
| (f) concentration critique de coagulation (CCC) à 20°C déterminée par mesure de turbidité en fonction de la salinité (NaCl). | | | | | |

En fin de polymérisation les particules sont collectées par simple centrifugation et redispersées dans l'eau ou dans un milieu désiré.

Les caractéristiques du polymère obtenu selon l'une quelconque des techniques 1) à 3) sont les suivantes :
- densité de charge (cationique) entre 5 et 150 µmol/g de polymère
- intervalle de la taille des particules comprise entre 0,05 et 2 µm, diamètre des particules mesuré par diffusion dynamique de la lumière à 20°C
- intervalle de la concentration critique de coagulation (CCC) entre 0,001 et 1,5 mole/l NaCl à 20°C et entre 0,01 et 0,9 mole/l NaCl à 40°C.

### EXEMPLE 2: ADSORPTION D'ARN OU DE BSA (BERUMALBUMINE BOVINE) SUR DES PARTICULES DE POLYMÈRE PNIPAM TELLES QUE PREPARÉES SELON L'EXEMPLE 1

Le protocole suivant constitue le mode opératoire général des réactions d'adsorption:

Le mélange réactionnel est constitué de 10 µl d'ARN (4 mg/ml) ou de 50 µl de BSA (5 mg/ml), et de 50 µl de particules NIPAM (45g/l). Le volume final de un millilitre est obtenu par adjonction de tampon phosphate (10 mM pH 4,6 ou 9,2) et NaCl (5M) afin d'atteindre le pH et la force ionique désirée.

L'entité moléculaire (ARN ou BSA) est adsorbée sur les particules durant 2 heures (à 20 ou 40°C) avec des conditions prédéterminées (pH, force ionique): le mélange est centrifugé 20 minutes à 14 000 tours par minute. Le surnageant est récupéré, filtré sur filtre Millipore Millex-GV13 (0,22 µm) afin d'éliminer les particules de polymère en suspension. La quantité de l'entité biologique fixée sur le support polymère est déterminée par une simple différence entre la quantité initialement introduite et la quantité restante et libre (dosée dans le surnageant): cette quantité est exprimée en milligramme de molécules biologiques par milligramme de polymère (Ns). Les concentrations d'ARN ou de BSA sont estimées par spectrophotométrie UV (Kontron Instrument) à une longueur d'onde de 260 nm ou 280 nm, respectivement.

Les essais ont été réalisés avec de l'ARN 16S et 23S ribosomal de *E. coli* (Boerhinger) et de la BSA (Sigma référence A0281) utilisés sans purification préalable.

Les particules utilisées sont des particules thermosensibles de PNIPAM94. Ces particules sont trés hydrophiles à température ambiante et hydrophobes à une température supérieure à la LCST (32°C). Elles ont été synthétisées comme décrit dans l'exemple 1.

Des tampons phosphate acide (KH₂PO₄ 10 mM pH 4,6) et basique (K₂HPO₄ 10 mM pH 9,2) ont été utilisés pour les réactions d'adsorption et pour contrôler le pH des réactions.

NaCl (5M) a été utilisé pour contrôler la force ionique des réactions.

L'eau utilisée dans l'ensemble des réactions a été purifiée sur le système de purification Millipore-Milli Q.

Les incubations ont été réalisées sur un thermomixer (Eppendorf 5436).

Toutes les réactions ont été réalisées dans des tubes Eppendorf de 1,5 ml.

### 1) Etude de l'influence du pH et de la température sur l'adsorption

Conformément à la Figure 2, on observe une meilleure adsorption de l'ARN à pH acide qu'à pH basique. A pH acide les particules sont largement chargées positivement et les acides nucléiques chargés négativement se fixent sur les particules via des forces électrostatiques. La fixation est plus importante à 20°C qu'à 40°C. Les résultats à 40°C illustrent une diminution de l'adsorption.

Conformément à la Figure 3, à 40 °C l'adsorption de la BSA sur les particules est possible sans influence du pH. A 20°C on n'observe aucune fixation de BSA en raison du caractère hydrophile des particules à cette température.

### 2) Etude de l'influence de la force ionique et de la température sur l'adsorption

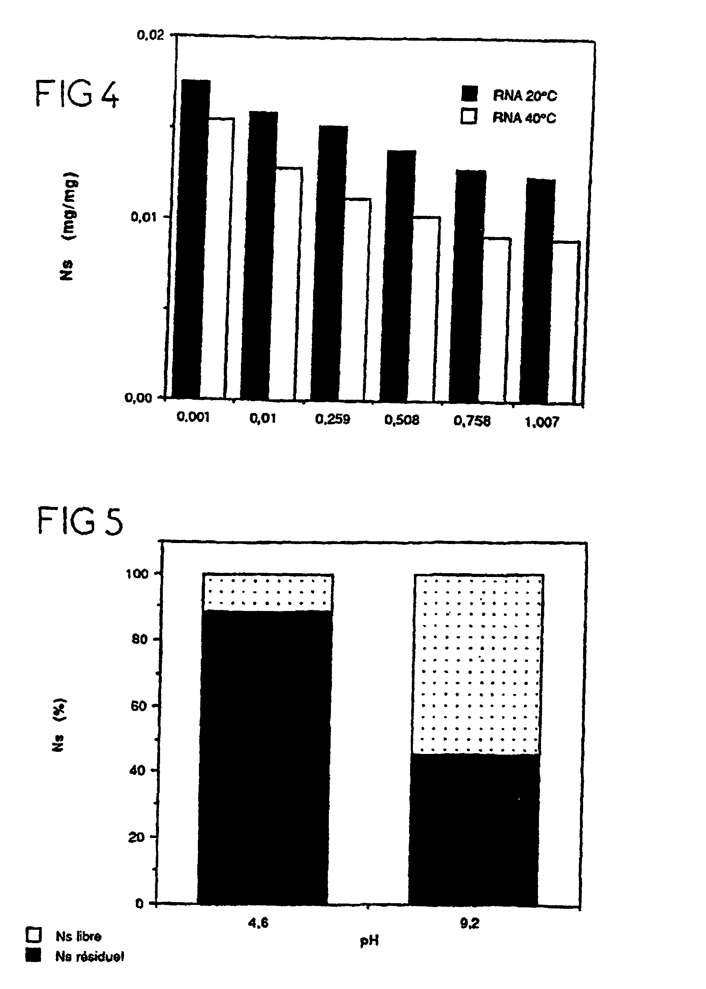

Conformément à la Figure 4, les forces électrostatiques attractives entre les ARN chargés négativement et la surface de polymère chargée positivement diminuent avec l'augmentation de la force ionique avec comme conséquence une diminution de la fixation de l'ARN.

Dans les mêmes conditions expérimentales, il a été vérifié que l'augmentation de la force ionique ne favorise pas la fixation de la BSA sur les particules.

En conclusion, les acides nucléiques sont préférentiellement adsorbés sur les particules à température inférieure à la LCST (20°C), à faible force ionique et pH acide. Dans ces conditions l'adsorption des protéines (telle la BSA) n'est pas favorisée.

### EXEMPLE 3: DÉSORPTION D'ARN ADSORBÉ SUR DES PARTICULES DE POLYMÈRE PNIPAM

Les réactifs utilisés sont les mêmes que ceux décrits dans l'exemple 2.

Le protocole suivant constitue le mode opératoire général des réactions de désorption:

Après une étape d'adsorption réalisée comme dans l'exemple 2, la réaction de désorption est effectuée après l'étape de centrifugation à 14000 tours par minute. le surnageant est éliminé et remplacé par un millilitre de tampon de désorption (phosphate (10mM pH 4,6 ou 9,2) et NaCl (5M)) afin d'atteindre le pH et la force ionique désirée. La désorption est réalisée pendant 2 heures à 20°C ou 40°C. Le mélange est ensuite centrifugé 20 minutes à 14000 tours par minute. Le surnageant est récupéré, filtré sur filtre Millipore Millex-GV13 (0,22 µm) afin d'éliminer les particules de polymère en suspension. La quantité d'ARN libérée est déterminée par spectrophotométrie UV (Kontron Instrument) à une longueur d'onde de 260 nm. L'acide nucléique récupéré est disponible pour d'autres analyses.

### 1) Etude de l'influence du pH et de la température sur la désorption de l'ARN

Conformément à la Figure 5, la désorption des acides nucléiques à pH basique est plus importante en raison de la perte de charge sur le polymère; à pH acide la quantité d'acides nucléiques libérés est beaucoup plus faible car les particules sont alors fortement chargées positivement.

Conformément à la Figure 6, comme précédemment la désorption des acides nucléiques est favorisée à pH basique. Elle est également favorisée par l'augmentation de la température car pour une température supérieure à la LCST (32°C) les particules se rétractent.

### 2) Etude de l'influence de la force ionique sur la désorption de l'ARN

Conformément à la Figure 7, au fur et à mesure de l'augmentation de la force ionique, les interactions électrostatiques attractives entre les ARN et la surface du polymère diminuent.

En conclusion, la désorption des acides nucléiques est préférentiellement réalisée à 40°C, à forte force ionique et pH basique.

Par ailleurs, la propriété de rétraction des particules à 40°C (température supérieure à la LCST) peut être exploitée pour concentrer une solution d'acide nucléique. En effet, après adsorption des acides nucléiques et élévation de la température au-delà de la LCST, les particules sur lesquelles sont adsorbés les acides nucléiques se rétractent, occupant ainsi un volume moindre qu'à l'état relaxé et permettant la reprise des particules, après centrifugation, dans un volume final plus faible.

### EXEMPLE 4: ADSORPTION ET DÉSORPTION D'ADN À PARTIR D'UNE SOLUTION MIXTE D'ADN ET DE BSA, EN UTILISANT LES PARTICULES DE NIPAM

La solution d'ADN de *Staphylococcus epidermidis* est extraite et purifiée à partir de colonies isolées de bactéries, selon le protocole décrit par D. TRECO dans Short Protocols in Molecular Biology Second Edition Ed : Harvard Medical School, 1992, pp 2-4/2-7.

Une solution de BSA (serum bovine albumine) (Intergen 3210-01) 10 % (p/v) en eau milliQ est utilisée.

Protocole PCR: la technique de PCR suivie est celle décrite par Goodman dans PCR Strategies Ed : Innis, Gelfand et Sninsky Academic Press 1995, pp17-31 Deux amorces d'amplification ont été utilisées; elles présentent les séquences suivantes:

Amorce 1 : 5' ATCTTGACATCCTCTGACC 3'--->SEQ ID NO1

Amorce 2 : 5' TCGACGGCTAGCTCCAAAT 3'--->SEQ ID NO2

Les cycles de température suivants ont été utilisés lors du protocole d'amplification:

| | | |
|---|---|---|
| 1 fois | 3 minutes | 94°C |
| | 2 minutes | 65°C |
| 35 fois | 1 minute | 72°C |
| | 1 minute | 94°C |
| | 2 minutes | 65°C |
| 1 fois | 5 minutes | 72°C |

10 µl de produit d'amplification sont déposés sur gel d'agarose 0,8% (FMC 50003) préalablement coloré au bromure d'éthidium. Après migration électrophorétique 45 minutes à 180V, les bandes d'acides nucléiques sont visualisées sous rayonnement ultra-violet (D. VOYTAS dans Short Protocols in Molecular Biology Second Edition Ed : Harvard Medical School, 1992, pp2-13/2-14).

### 1) Adsorption et désorption d'ADN sur les particules et détection après technique PCR, de l'ADN libéré

Une solution d'ADN (10¹⁰ copies/ml) a été adsorbée sur les particules à 20°C, pH 4,6 pendant deux heures puis soumise à une étape de désorption de 15 minutes à 41°C, pH 8,3, force ionique 0,05 M comme décrit dans les exemples 2 et 3, respectivement. Après l'étape de désorption et centrifugation, le matériel récupéré dans 50 µl de surnageant a été amplifié par PCR et analysé sur gel d'agarose 0,8 %. Une bande de taille attendue (490 pb) est détectée sur gel. Par ailleurs, la quantité d'ADN détectée après PCR est au moins équivalente à celle détectée après amplification par PCR de 10⁶ copies/ml d'ADN non adsorbé au préalable sur particules.

Les particules de NIPAM94 peuvent donc être également utilisées pour adsorber de l'ADN. Après désorption l'ADN peut être utilisé dans une réaction d'amplification de type PCR.

### 2) Adsorption d'ADN à partir d'une solution mixte ADN et BSA, et détection après technique PCR, de l'ADN libéré par désorption

Une solution d'ADN (10¹⁰ copies/ml) en présence de 10 % (p/v) de BSA est soumise à une étape d'adsorption et de désorption comme décrit dans l'exemple 4-1. Les mêmes techniques d'amplification et de détection sont utilisées. Un ADN de taille attendue (490 pb) est détecté sur gel. L'intensité de la bande d'ADN visualisée est la même en présence ou en absence de BSA.

Les particules de NIPAM94 permettent d'adsorber et de libérer par désorption de l'ADN provenant d'une solution mixte ADN - 10% BSA. La présence de la BSA dans la solution initiale ne perturbe pas l'adsorption de l'ADN sur les particules.

### EXEMPLE 5 : PURIFICATION D'ACIDES NUCLEIQUES ISSUS D'UN LYSAT BACTERIEN (Staphylococcus epidermidis) EN UTILISANT LES PARTICULES DE NIPAM

### 1) Préparation du lysat bactérien

Une culture de *Staphylococcus epidermidis* est réalisée pendant une nuit à 37°C. Le nombre de bactéries contenues dans la suspension est estimé par mesure de la densité optique à 550 nm. Des culots bactériens, contenant respectivement 2.10⁶, 2.10⁴ et 2.10¹ bactéries, sont réalisés en tubes de 1,5 ml par centrifugation 3 minutes à 14 000 tours. Le surnageant est éliminé et le culot bactérien est lysé selon la technique décrite ci-dessous (adaptation de Arora et al., J. Dairy Sci. 1990, 73, 264-273).

Le culot est repris par 1 ml de tampon (Tris 30 mM, NaCl 100 mM, EDTA 5 mM, pH 7,2) contenant 6 mg/ml de protéinase K (Boehringer) et 300 µl de billes de verre. Ce mélange est agité sur un vortex et incubé 15 minutes à 37°C. Après une étape de centrifugation (3 minutes à 14 000 tours), le surnageant, contenant les acides nucléiques est récupéré pour les étapes ultérieures.

### 2) Purification des acides nucléiques

Les particules utilisées sont des particules thermosensibles de PNIPAM94 dont la synthèse est décrite dans l'exemple 1.

Le protocole suivant constitue le mode opératoire général des réactions de purification.

Le mélange réactionnel est constitué de 50 µl de lysat bactérien, contenant respectivement 10⁵, 10³ et 10⁶ bactéries, et de 2 mg de particules. Le volume final de un millilitre est obtenu en complétant le volume réactionnel par du tampon phosphate (10 mM, pH 4,6). La réaction est incubée durant 30 minutes sur un thermomixer (Eppendorf 5436) à 20°C. Après une étape de centrifugation de 20 minutes, à 14 000 tours par minute, le surnageant est éliminé. La désorption des acides nucléiques, fixés sur les particules, est réalisée par l'effet de la force ionique en ajoutant 50 µl de tampon d'élution (KCl 0,5 M, pH 8,3) ; la réaction est incubée durant 15 minutes à 42°C sur le thermomixer. Après une nouvelle étape de centrifugation de 20 minutes, à 14 000 tours par minute, le surnageant contenant les acides nucléiques est récupéré ; 10 µl sont utilisés pour une étape d'amplification de l'ADN (PCR) et 5 µl pour une étape d'amplification de l'ARN (NASBA).

### 3) Détection des acides nucléiques

Les acides nucléiques purifiés sont analysés après une étape d'amplification enzymatique (PCR pour ADN et NASBA pour ARN). Les produits d'amplification sont ensuite révélés par des techniques ELOSA (Enzyme Linked Oligo Sorbent Assay) en microplaque (NASBA) ou VIDAS (PCR).

Protocole PCR : le protocole suivi est le même que celui décrit dans l'exemple 4. Les produits d'amplification (90 µl) sont analysés sur l'automate d'immuno-analyse Vidas (bioMérieux) conformément au protocole décrit par Mabilat et al., J. Clin. Microbiol ; 1994, 32, 2702-2705, les sondes de capture et de détection étant les suivantes :
sonde de capture : 5' ACCACCTGTCACTCTGTCCC 3' SEQ ID NO: 3
sonde de détection :

5' GGAAGGGGAAAACTCTATCTC 3' SEQ ID NO: 4

La sonde de détection est conjuguée à la phosphatase alcaline.

Protocole NASBA : le protocole suivi est le même que celui décrit par Kievits et al., J. Virol. Methods (1991) 35, 273-286. Les amorces utilisées présentent les séquences suivantes :
amorce 1 : 5' TCGAAGCAACGCGAAGAACCTTACCA 3' SEQ ID NO: 5
amorce 2 :

5' AATTCTAATA CGACTCACTA TAGGGAGGTT TGTCACCGGC AGTCAACTTA
GA 3' SEQ ID NO: 6

Les produits d'amplification (5 µl) sont analysés avec une technique Elosa en format microplaque conformément au protocole décrit par Mallet et al., J. Clin. Microbiol. (1993) 31, 1444-1449. Les sondes de capture et de détection présentent les séquences suivantes :
sonde de capture : 5' GATAGAGTTTTCCCCTTC 3' SEQ ID NO: 7
sonde de détection :

5' GACATCCTCTGACCCCTCTA 3' SEQ ID NO: 8

La sonde de détection est conjuguée à la péroxydase de raifort.

La protéinase K étant un inhibiteur connu des réactions d'amplification, des dilutions. de 1/10 en 1/10, sont réalisées avant les étapes d'amplification pour quantifier le degré de purification obtenu.

Les résultats obtenus sont rassemblés dans le tableau IV en annexe.

On vérifie le pouvoir inhibiteur de la protéinase K puisqu'il faut diluer l'échantillon au 1/1 000 avant l'étape d'amplification. Après l'étape de purification l'échantillon n'est plus dilué qu'au 1/10 avant l'étape d'amplification, ce qui représente un gain d'un facteur 100. Les particules permettent de purifier conjointement l'ARN et l'ADN présents dans l'échantillon. Ces acides nucléiques sont compatibles avec les étapes d'amplification enzymatique.

### EXEMPLE 6 : PURIFICATION D'ACIDES NUCLEIQUES ISSUS D'UN LYSAT BACTERIEN (Staphylococcus epidermidis) EN UTILISANT LE POLYMERE NIPAM GREFFE SUR UN NOYAU MAGNETIQUE

Les particules décrites dans les exemples précédents présentent l'inconvénient de nécessiter des étapes de centrifugation après les étapes d'adsorption et de désorption. Ces étapes sont longues (2 fois 20 minutes) et difficilement automatisables. Une alternative possible est de greffer le polymère de Nipam sur des supports magnétiques cationiques. Un des supports testés est le latex magnétique cationique R95-07 (Estapor, Rhône-Poulenc) dont les particules sont polydisperses.

La capacité de purification des particules ainsi obtenues a été testée.

### 1) Synthèse des particules magnétiques de Nipam

Les particules Estapor cationiques R95-07 ont été encapsulées. Avant chaque encapsulation les particules ont été lavées 3 fois avec une solution d'acide chlorhydrique 0,005 M.

5 1 g de particules semence est dilué dans 40 ml d'eau milliQ préalablement chauffée à ébullition et dégazée avec de l'azote.
Styrène : 100 µg
NIPAM : 0,3254 g
BAM : 0,0274 g
MAE : 0,0740 g
Triton X-405 : 0,14 g
V50 : 0,0061 g

100 µg du styrène pour l'étape de prégonflement (temps 2 h à 70°C), le NIPAM, BAM et MAE sont solubilisés dans 10 ml d'eau et introduits sur la semence (latex Estapor). L'amorceur solubilisé dans 1 ml d'eau, est ajouté pour permettre la polymérisation autour des particules semence. La polymérisation a été réalisée sous atmosphère d'azote, à 70°C.

Ces particules portent alors une charge de 220 et de 82 µmol de NH₂/g de particules sans modifier la distribution en taille des particules.

### 2) Purification des acides nucléiques

Le protocole utilisé est le même que celui décrit dans l'exemple 5 avec les modifications suivantes.
- 200 µg de particules ont été utilisées,
- les étapes de centrifugation, pour séparer les particules des surnageants, sont supprimées et remplacées par des étapes de séparation sous l'effet d'un champ magnétique (dispositif de séparation magnétique, Promega® Z5342).

L'ensemble des autres étapes demeure inchangé.

Les résultats sont rassemblés dans le tableau V en annexe.

On retrouve le pouvoir inhibiteur de la protéinase K puisqu'il faut diluer l'échantillon au 1/1 000 avant l'étape d'amplification. Après l'étape de purification, l'échantillon peut être dilué au 1/10 (PCR) ou 1/100 (NASBA) avant l'étape de purification, ce qui représente un gain d'un facteur 10 à 100. Ces particules permettent également de purifier conjointement l'ARN et l'ADN présent dans l'échantillon. Ces acides nucléiques sont compatibles avec les étapes d'amplification enzymatique.

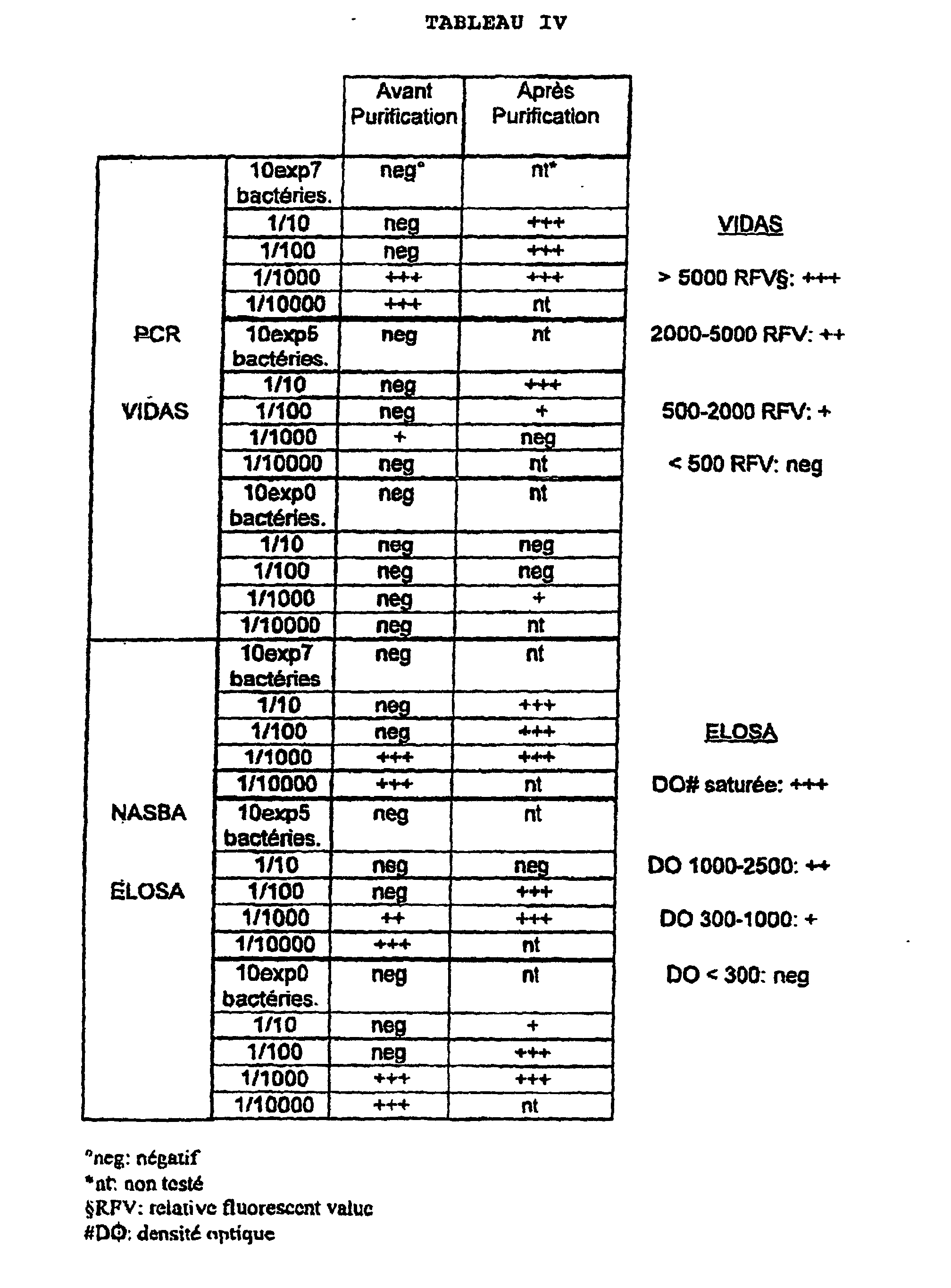

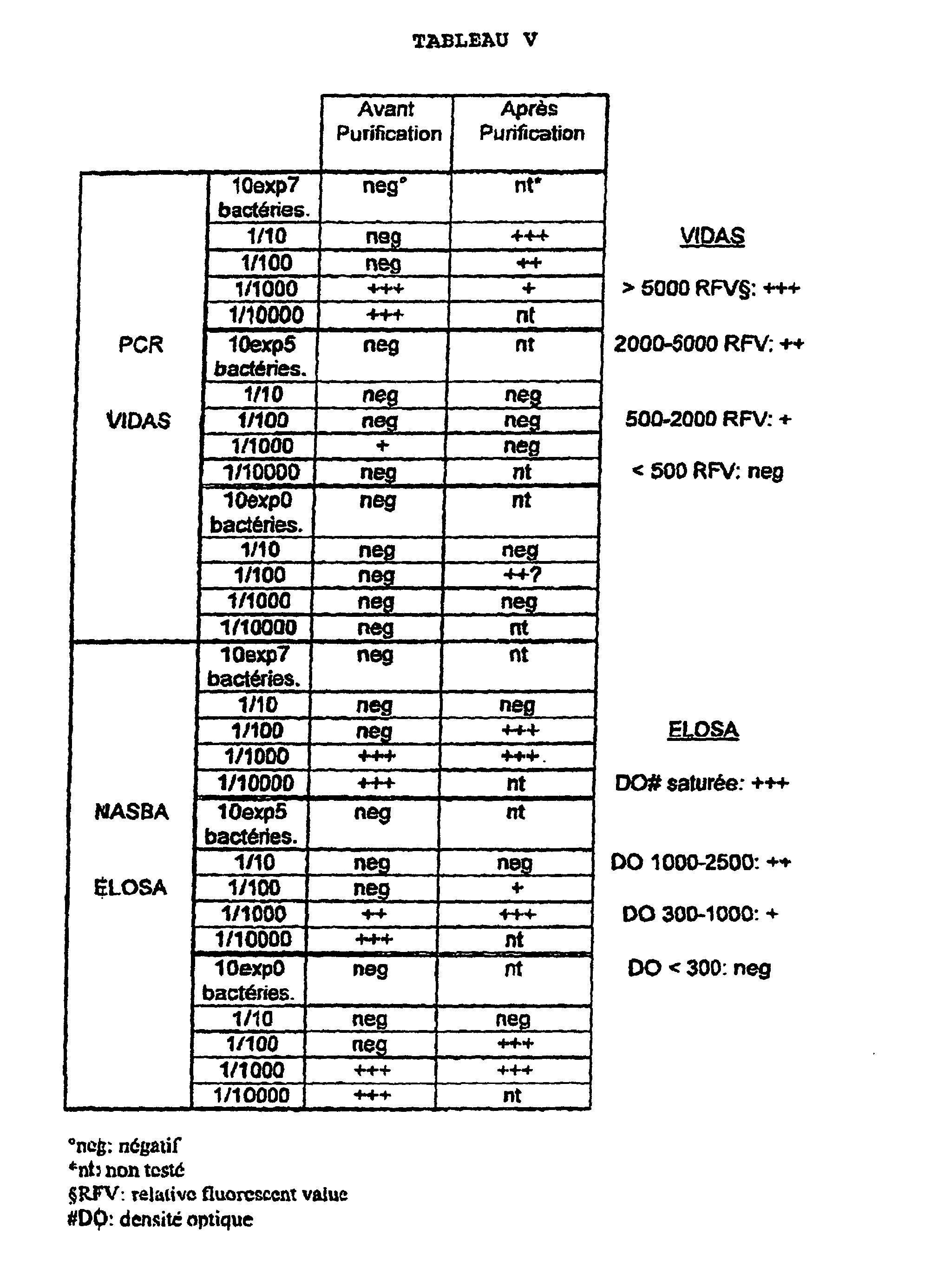

## Revendications

1. Procédé d'isolement, en phase aqueuse, d'un matériel nucléique, présent dans un échantillon, comprenant une étape d'adsorption dudit matériel nucléique, sur un support particulaire, **caractérisé en ce que** :
* selon une étape (a) dite d'obtention du réactif d'adsorption, on dispose d'un réactif d'adsorption comprenant un sol constitué par une phase continue aqueuse et une phase discontinue du support particulaire qui comprend un polymère particulaire, fonctionnalisé, ledit polymère étant obtenu par polymérisation de (1) un premier monomère hydrosoluble, d'acrylamide ou d'un dérivé d'acrylamide, (2) au moins un agent de réticulation et (3) au moins un second monomère, fonctionnel, cationique et hydrosoluble, et ledit polymère présentant une température critique inférieure de solubilité (LCST) prédéterminée qui est comprise entre 25 et 45°C,
* selon une étape (b) dite de mise en contact, on met en contact le réactif d'adsorption avec l'échantillon contenant le matériel nucléique,
* selon une étape (c) dite d'adsorption, pour la mise en contact selon (b), on choisit au moins un des paramètres suivants pour le milieu réactionnel:
- pH au plus égal à 7,
- force ionique au plus égale à 10⁻² M,
- température inférieure à la LCST du polymère,
* selon une étape (d) dite de séparation, après éventuellement avoir observé que l'adsorption a eu lieu, on sépare la phase discontinue de la phase continue, et
* selon une étape (e) dite de désorption, on dissocie, par désorption, le matériel nucléique par rapport au support particulaire, en augmentant la force ionique jusqu'à une force ionique supérieure à 10⁻²M.

2. Procédé selon la revendication 1, **caractérisé en ce que** pour l'étape (e) de désorption, on fait en outre varier au moins un des paramètres choisis parmi le pH et la température comme suit :
- augmentation du pH jusqu'à un pH supérieur à 7,
- augmentation de la température jusqu'à une température supérieure à la LCST du polymère.

3. Procédé d'isolement, en phase aqueuse, d'un matériel nucléique, présent dans un échantillon, comprenant une étape d'adsorption dudit matériel nucléique, sur un support particulaire, **caractérisé en ce que** :
* selon une étape (a) dite d'obtention du réactif d'adsorption, on dispose d'un réactif d'adsorption comprenant un sol constitué par une phase continue aqueuse et une phase discontinue du support particulaire qui comprend un polymère particulaire, fonctionnalisé, ledit polymère étant obtenu par polymérisation de (1) un premier monomère hydrosoluble, d'acrylamide ou d'un dérivé d'acrylamide, (2) au moins un agent de réticulation et (3) au moins un second monomère, fonctionnel, cationique et hydrosoluble, et ledit polymère présentant une température critique inférieure de solubilité (LCST) prédéterminée qui est comprise entre 25 et 45°C,
* selon une étape (b) dite de mise en contact, on met en contact le réactif d'adsorption avec l'échantillon contenant le matériel nucléique,
* selon une étape (c) dite d'adsorption, on choisit, pour la mise en contact selon (b), une force ionique au plus égale à 10⁻² M pour le milieu réactionnel,
* selon une étape (d) dite de séparation, après éventuellement avoir observé que l'adsorption a eu lieu, on sépare la phase discontinue de la phase continue.

4. Procédé selon la revendication 3, **caractérisé en ce que**, selon l'étape (c) d'adsorption, on choisit en outre, pour la mise en contact selon (b), au moins un des paramètres suivants pour le milieu réactionnel :
- pH au plus égal à 7,
- température inférieure à la LCST du polymère.

5. Procédé selon l'une quelconque des revendications 1 à 4, **caractérisé en ce que** le support particulaire consiste en un polymère particulaire, fonctionnalisé, obtenu par polymérisation de (1) un premier monomère hydrosoluble, d'acrylamide ou d'un dérivé d'acrylamide, (2) au moins un agent de réticulation hydrosoluble et (3) au moins un second monomère, fonctionnel, cationique et hydrosoluble, et ledit polymère présentant une température critique inférieure de solubilité (LCST) prédéterminée qui est comprise entre 25 et 45°C.

6. Procédé selon l'une quelconque des revendications 1 à 4, **caractérisé en ce que** le support particulaire comprend en outre un noyau magnétique, recouvert en totalité ou en partie par ledit polymère particulaire, ledit noyau ne modifiant pas les propriétés d'adsorption du polymère vis-à-vis dudit matériel nucléique.

7. Procédé selon la revendication 6, **caractérisé en ce que** le noyau est un noyau magnétique de polystyrène.

8. Procédé selon l'une quelconque des revendications précédentes, **caractérisé en ce qu'**on ajoute dans l'échantillon avant l'étape (b), ou dans le milieu réactionnel après l'étape (b) et notamment après l'étape (c) ou l'étape (d), au moins une sonde et/ou une amorce susceptible de s'hybrider spécifiquement sur le matériel nucléique.

9. Procédé d'isolement, en phase aqueuse, d'un matériel nucléique, présent dans un échantillon, **caractérisé en ce que** :
* selon une étape (a) dite d'obtention du réactif d'adsorption, on dispose d'un réactif d'adsorption comprenant un sol constitué par une phase continue aqueuse et une phase discontinue du support particulaire qui comprend un polymère particulaire, fonctionnalisé, ledit polymère étant obtenu par polymérisation de (1) un premier monomère hydrosoluble, d'acrylamide ou d'un dérivé d'acrylamide, (2) au moins un agent de réticulation et (3) au moins un second monomère, fonctionnel, cationique et hydrosoluble, et ledit polymère présentant une température critique inférieure de solubilité (LCST) prédéterminée qui est comprise entre 25 et 45°C,
* selon une étape (b) dite de mise en contact, on met en contact le réactif d'adsorption avec une sonde ou une amorce,
* selon une étape (c) dite d'adsorption, pour la mise en contact selon (b), on choisit au moins un des paramètres suivants pour le milieu réactionnel:
- pH au plus égal à 7,
- force ionique au plus égale à 10⁻² M,
- température inférieure à la LCST du polymère, pour obtenir un réactif d'hybridation,
* selon une étape (d) dite de séparation, après éventuellement avoir observé que l'adsorption a eu lieu, on sépare la phase discontinue de la phase continue,
* selon une étape (b') dite d'obtention d'un complexe d'hybridation, on met en contact le réactif d'hybridation avec ledit matériel nucléique, et
* selon une étape (e) dite de désorption, on dissocie, par désorption, le complexe d'hybridation par rapport au support particulaire, en augmentant la force ionique jusqu'à une force ionique supérieure à 10⁻²M.

10. Procédé selon l'une quelconque des revendications précédentes, **caractérisé en ce que** la LCST du polymère est comprise entre 30 et 40°C.

11. Procédé selon l'une quelconque des revendications précédentes, **caractérisé en ce que** le premier monomère (1) est chois parmi les N-alkylacrylamides et les N,N-dialkylacrylamides.

12. Procédé selon la revendication 11, **caractérisé en ce que** le premier monomère (1) est choisi parmi le N-isopropylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacrylamide, le premier monomère étant de préférence le N-isopropylacrylamide (NIPAM).

13. Procédé selon l'une quelconque des revendications précédentes, **caractérisé en ce que** le ou les seconds monomères fonctionnels (3) sont choisis parmi les dérivés acryliques et méthacryliques, le chlorure de 2-aminoéthyl méthacrylate (AEM), les dérivés de N-vinyl-pyridine, les dérivés de trialkylammonium et les dérivés de chlorure d'isothiouronium.

14. Procédé selon l'une quelconque des revendications précédentes, **caractérisé en ce que** l'agent de réticulation hydrosoluble (2) est choisi parmi le N,N-méthylène bisacrylamide (MBA), l'éthylène glycol diméthacrylate.

15. Procédé selon l'une quelconque des revendications précédentes, **caractérisé en ce que** selon l'étape (a), ledit polymère est obtenu par polymérisation radicalaire en présence d'un amorceur de polymérisation choisi parmi les amorceurs neutres et cationiques, hydrosolubles, tel que le chlorure de 2,2'-azobis amidino-propane (V50).

16. Procédé selon la revendication 3, **caractérisé en ce qu'**il comprend, après l'étape (d) de séparation, une étape dite de désorption, selon laquelle on dissocie, par désorption, le matériel nucléique par rapport au support particulaire, en faisant varier au moins un des paramètres choisis parmi la force ionique, le pH et la température, comme suit :
- augmentation de la force ionique jusqu'à une force ionique supérieure à 10⁻²M,
- augmentation du pH jusqu'à un pH supérieur à 7,
- augmentation de la température jusqu'à une température supérieure à la LCST du polymère.

17. Procédé selon l'une quelconque des revendications précédentes, **caractérisé en ce que** l'étape (d) de séparation est effectuée par une technique choisie parmi la centrifugation, la filtration, la précipitation, la sédimentation, et l'application d'un champ magnétique.

## Claims

1. Process for the isolation in aqueous phase of a nucleic material present in a sample, comprising a step of adsorption of said nucleic material onto a particulate support, **characterized in that** :
* according to a so-called step (a) for producing the adsorption reagent, an adsorption reagent is available which comprises a sol consisting of an aqueous continuous phase and a discontinuous phase of the particulate support which comprises a functionalized, particulate polymer, said polymer being obtained by polymerization of (1) a first water-soluble monomer of acrylamide or of an acrylamide derivative, (2) at least one cross-linking agent and (3) at least a second cationic and water-soluble functional monomer, said polymer having a predetermined lower critical solubility temperature (LCST) which is between 25 and 45°C,
* according to a so-called step (b) for bringing into contact, the adsorption reagent is brought into contact with the sample containing the nucleic material,
* according to a so-called adsorption step (c), for the bringing into contact according to (b),at least one of the following parameters for the reaction medium is chosen :
- pH at most equal to 7,
- ionic strength at most equal to 10⁻² M,
- temperature less than the LCST of the polymer,
* according to a so-called separation step (d), after having optionally observed that the adsorption has taken place, the discontinuous phase is separated from the continuous phase, and
* according to a so-called desorption step (e), the nucleic material is dissociated, by desorption, from the particulate support by increasing the ionic strength up to an ionic strength greater than 10⁻² M.

2. Process according to Claim 1, **characterized in that** for the desorption step (e), at least one of the parameters selected from the pH and the temperature is in addition varied as follows:
- increase in the pH up to a pH greater than 7,
- increase in the temperature up to a temperature greater than the LCST of the polymer.

3. Process for the isolation in aqueous phase of a nucleic material present in a sample, comprising a step of adsorption of said nucleic material onto a particulate support, **characterized in that** :
* according to a so-called step (a) for producing the adsorption reagent, an adsorption reagent is available which comprises a sol consisting of an aqueous continuous phase and a discontinuous phase of the particulate support which comprises a functionalized, particulate polymer, said polymer being obtained by polymerization of (1) a first water-soluble monomer of acrylamide or of an acrylamide derivative, (2) at least one cross-linking agent and (3) at least a second cationic and water-soluble functional monomer, said polymer having a predetermined lower critical solubility temperature (LCST) which is between 25 and 45°C,
* according to a so-called step (b) for bringing into contact, the adsorption reagent is brought into contact with the sample containing the nucleic material,
* according to a so-called adsorption step (c), for the bringing into contact according to (b), an ionic strength at most equal to 10⁻² M is selected for the reaction medium,
* according to a so-called separation step (d), after having optionally observed that the adsorption has taken place, the discontinuous phase is separated from the continuous phase.

4. Process according to Claim 3, **characterized in that**, according to the adsorption step (c), for the bringing into contact according to (b), at least one of the following parameters is in addition selected for the reaction medium :
- pH at most equal to 7,
- temperature less than the LCST of the polymer.

5. Process according to any one of Claims 1 to 4, **characterized in that** the particulate support consists of a functionalized particulate polymer obtained by polymerization of (1) a first water-soluble monomer of acrylamide or of an acrylamide derivative, (2) at least one water-soluble cross-linking agent and (3) at least a second cationic and water-soluble functional monomer, said polymer having a predetermined lower critical solubility temperature (LCST) which is between 25 and 45°C.

6. Process according to any one of Claims 1 to 4, **characterized in that** the particulate support comprises, in addition, a magnetic core, completely or partially coated with said particulate polymer, said core not modifying the adsorption properties of the polymer in relation to said nucleic material.

7. Process according to Claim 6, **characterized in that** the core is a polystyrene core.

8. Process according to any one of the preceding claims, **characterized in that** at least one probe and/or one primer capable of specifically hybridizing to the nucleic material is added to the sample before step (b), or to the reaction medium after step (b), and in particular after step (c) or step (d).

9. Process for the isolation in aqueous phase of a nucleic material present in a sample, **characterized in that** :
* according to a so-called step (a) for producing the adsorption reagent, an adsorption reagent is available which comprises a sol consisting of an aqueous continuous phase and a discontinuous phase of the particulate support which comprises a functionalized, particulate polymer, said polymer being obtained by polymerization of (1) a first water-soluble monomer of acrylamide or of an acrylamide derivative, (2) at least one cross-linking agent and (3) at least a second cationic and water-soluble functional monomer, said polymer having a predetermined lower critical solubility temperature (LCST) which is between 25 and 45°C,
* according to a so-called step (b) for bringing into contact, the adsorption reagent is brought into contact with a probe or a primer,
* according to a so-called adsorption step (c), for bringing into contact according to (b),at least one of the following parameters for the reaction medium is chosen :
- pH at most equal to 7,
- ionic strength at most equal to 10⁻² M,
- temperature less than the LCST of the polymer,
* according to a so-called separation step (d), after having optionally observed that the adsorption has taken place, the discontinuous phase is separated from the continuous phase,
* according to a so-called step (b') for producing the hybridization complex, the hybridization reagent is brought into contact whith said nucleic material, and
* according to a so-called desorption step (e), the hybridization complex is dissociated, by desorption, from the particulate support by increasing the ionic strength up to an ionic strength greater than 10⁻² M.

10. Process according to any one of the preceding claims, **characterized in that** the LCST of the polymer is between 30 and 40°C.

11. Process according to any one of the preceding claims, **characterized in that** the first monomer (1) is selected from N-alkylacrylamides and N,N-dialkylacrylamides.

12. Process according to Claim 11, **characterized in that** the first monomer (1) is selected from N-isopropyiacrylamide, N-ethylmethacrylamide, N-n propylacrylamide, N-n-propylmethacrylamide, N-isopropylmethacrylamide, N-cyclopropylacrylamide, N,N-diethylacrylamide, N-methyl-N-isopropylacrylamide, N-methyl-N-n-propylacrylamide, the first monomer being preferably N-isopropylacryiamide (NIPAM).

13. Process according to any one of the preceding claims, **characterized in that** the second functional monomer(s) (3) are selected from the acrylic and methacrylic derivatives, 2-aminoethylmethacrylate chloride (AEM), the N-vinylpyridine derivatives, the trialkylammonium derivatives and the isothiouronium chloride derivatives.

14. Process according to any one of the preceding claims, **characterized in that** the water-soluble crosslinking agent (2) is selected from N,N-methylenebisacrylamide (MBA), ethylene glycol dimethacrylate.

15. Process according to any one of the preceding claims, **characterized in that** according to step (a), said polymer is obtained by radical polymerization in the presence of a polymerization initiator selected from the water-soluble neutral and cationic initiators such as 2,2'-azobisamidinopropane chloride (V50).

16. Process according to Claim 3, **characterized in that** it comprises, after the separation step (d), a so-called desorption step according to which the nucleic material is dissociated, by desorption, from the particulate support by varying at least one of the parameters selected from the ionic strength, the pH and the temperature, as follows :
- increase in the ionic strength up to an ionic strength greater than 10⁻²M,
- increase in the pH up to a pH greater than 7,
- increase in the temperature up to a temperature greater than the LCST of the polymer.

17. Process according to any one of the preceding claims, **characterized in that** the separation step (d) is performed by a technique selected from centrifugation, filtration, precipitation, sedimentation, and the application of a magnetic field.

## Patentansprüche

1. Verfahren, um in flüssiger Phase Nukleinsäurematerial zu isolieren, das in einer Probe vorhanden ist, mit einem Schritt der Adsorption des Nukleinsäurematerials an einem aus Partikeln bestehenden Träger, **dadurch gekennzeichnet, daß**:
* in einem Erhalt des Adsorptionsreagenz genannten Schritt (a) ein Adsorptionsreagenz bereitgestellt wird, das ein Sol aufweist, das durch eine wässrige kontinuierliche Phase und eine diskontinuierliche Phase des aus Partikeln bestehenden Trägers gebildet wird, der ein funktionalisiertes, aus Partikeln bestehendes Polymer umfaßt, wobei das Polymer durch Polymerisation von (1) einem ersten wasserlöslichen Monomer von Acrylamid oder einem Acrylamidderivat, (2) wenigstens einem vernetzungsreagenz und (3) wenigstens einem zweiten funktionellen, kationischen und wasserlöslichen Monomer erhalten wird, und das Polymer eine vorbestimmte untere kritische Löslichkeitstemperatur (LCST) aufweist, die zwischen 25 und 45°C liegt,
* in einem Inkontaktbringen genannten Schritt (b) das Adsorptionsreagenz mit der das Nukleinsäurematerial enthaltenden Probe in Kontakt gebracht wird,
* in einem Adsorption genannten Schritt (c) für das Inkontaktbringen gemäß (b) wenigstens einer der folgenden Parameter für das Reaktionsmedium ausgewählt wird:
- pH-Wert nicht höher als 7,
- Ionenstärke nicht höher als 10⁻² M,
- Temperatur unterhalb der LCST des Polymers,
* in einem Separation genannten Schritt (d) die diskontinuierliche Phase von der kontinuierlichen Phase getrennt wird, nachdem ggf. beobachtet wurde, daß die Adsorption stattgefunden hat, und
* in einem Desorption genannten Schritt (e) das Nukleinsäurematerial von dem aus Partikeln bestehenden Träger dissoziiert wird, indem die Ionenstärke bis auf eine Ionenstärke oberhalb von 10⁻² M erhöht wird,

2. Verfahren nach Anspruch 1, **dadurch gekennzeichnet, daß** für den Schritt (e) der Desorption u.a. wenigstens einer der Parameter pH-Wert oder Temperatur wie folgt variiert wird:
- Erhöhung des pH-Wertes bis auf einen pH-Wert oberhalb von 7,
- Erhöhung der Temperatur bis auf eine Temperatur oberhalb des LCST des Polymers.

3. Verfahren, um in flüssiger Phase Nukleinsäurematerial zu isolieren, das in einer Probe vorhanden ist, mit einem Schritt der Adsorption des Nukleinsäurematerials an einem aus Partikeln bestehenden Träger, **dadurch gekennzeichnet, daß**:
* in einem Erhalt des Adsorptionsreagenz genannten Schritt (a) ein Adsorptionsreagenz bereitgestellt wird, das ein Sol aufweist, das durch eine wässrige kontinuierliche Phase und eine diskontinuierliche Phase des aus Partikeln bestehenden Trägers gebildet wird, der ein funktionalisiertes, aus Partikeln bestehendes Polymer umfaßt, wobei das Polymer durch Polymerisation von (1) einem ersten wasserlöslichen Monomer von Acrylamid oder einem Acrylamidderivat, (2) wenigstens einem Vernetzungsreagenz und (3) wenigstens einem zweiten funktionellen, kationischen und wasserlöslichen Monomer erhalten wird, und das Polymer eine vorbestimmte untere kritische Löslichkeitstemperatur (LCST) aufweist, die zwischen 25 und 45°C liegt,
* in einem Inkontaktbringen genannten Schritt (b) das Adsorptionsreagenz mit der das Nukleinsäurematerial enthaltenden Probe in Kontakt gebracht wird,
* in einem Adsorption genannten Schritt (c) für das Inkontaktbringen gemäß (b) für das Reaktionsmedium eine Ionenstärke nicht höher als 10⁻² M gewählt wird,
* in einem Separation genannten Schritt (d) die diskontinuierliche Phase von der kontinuierlichen Phase getrennt wird, nachdem ggf. beobachtet wurde, daß die Adsorption stattgefunden hat.

4. Verfahren nach Anspruch 3, **dadurch gekennzeichnet, daß** für den Schritt (c) der Adsorption u.a. für das Inkontaktbringen gemäß (b) für das Reaktionsmedium wenigstens einer der folgenden Parameter ausgewählt wird:
- pH-Wert nicht höher als 7,
- Temperatur unterhalb der LCST des Polymers.

5. Verfahren nach einem der Ansprüche 1 bis 4, **dadurch gekennzeichnet, daß** der aus Partikeln bestehende Träger aus einem aus Partikeln bestehenden funktionalisierten Polymer besteht, das durch Polymerisation von (1) einem wasserlöslichen ersten Monomer von Acrylamid oder einem Aarylamidderivat, von (2) wenigstens einem wasserlöslichen Vernetzungsreagenz und von (3) wenigstens einem zweiten funktionellen, kationischen und wasserlöslichen Monomer erhalten wird, und das Polymer eine vorbestimmte untere kritische Löslichkeitstemperatur (LCST) aufweist, die zwischen 25 und 45°C liegt.

6. Verfahren nach einem der Ansprüche 1 bis 4, **dadurch gekennzeichnet, daß** der aus Partikeln bestehende Träger u.a. einen ganz oder teilweise mit dem aus Partikeln bestehenden Polymer bedeckten Kern umfaßt, wobei der Kern die Adsorptionseigenschaften des Polymers für das Nukleinsäurematerial nicht verändert.

7. Verfahren nach Anspruch 6, **dadurch gekennzeichnet, daß** der Kern ein magnetischer Kern aus Polystyrol ist.

8. Verfahren naoh einem der vorhergehenden Ansprüche, **dadurch gekennzeichnet, daß** der Probe vor dem Schritt (b) oder dem Reaktionsmedium nach dem Schritt (b) und insbesondere nach dem Schritt (c) oder dem Schritt (d) wenigstens eine Sonde und/oder ein Primer zugegeben wird, die/der spezifisch mit dem Nukleinsäurematerial hybridisieren kann.

9. Verfahren, um in flüssiger Phase Nukleinsäurematerial zu isolieren, das in einer Probe vorhanden ist, **dadurch gekennzeichnet, daß**:
* in einem Erhalt des Adsorptionsreagenz genannten Schritt (a) ein Adsorptionsreagenz bereitgestellt wird, das ein Sol aufweist, das durch eine wässrige kontinuierliche Phase und eine diskontinuierliche Phase des aus Partikeln bestehenden Trägers gebildet wird, der ein funktionalisiertes, aus Partikeln bestehendes Polymer umfaßt, wobei das Polymer durch Polymerisation von (1) einem ersten wasserlöslichen Monomer von Acrylamid oder einem Acrylamidderivat, (2) wenigstens einem Vernetzungsreagenz und (3) wenigstens einem zweiten funktionellen, kationischen und wasserlöslichen Monomer erhalten wird, und das Polymer eine vorbestimmte untere kritische Löslichkeitstemperatur (LCST) aufweist, die zwischen 25 und 45°C liegt,
* in einem Inkontaktbringen genannten Schritt (b) das Adsorptionsreagenz mit einer Sonde oder einem Primer in Kontakt gebracht wird,
* in einem Adsorption genannten Schritt (c) für das Inkontaktbringen gemäß (h) für das Reaktionsmedium wenigstens einer der folgenden Parameter ausgewählt wird:
- pH-Wert nicht höher als 7,
- Ionenstärke nicht höher als 10⁻² M,
- Temperatur unterhalb der LCST des Polymers, um ein Hybridisierungsreagenz zu erhalten,
* in einem Separation genannten Schritt (d) die diskontinuierliche Phase von der kontinuierlichen Phase getrennt wird, nachdem ggf. beobachtet wurde, daß die Adsorption stattgefunden hat,
* in einem Erhalt eines Hybridisierungskomplexes genannten Schritt (b') das Hybridisierungsreagenz mit dem Nukleinsäurematerial in Kontakt gebracht wird, und
* in einem Desorption genannten Schritt <e) der Hybridisierungskomplex durch Desorption von dem aus Partikeln bestehenden Träger dissoziiert wird, indem die Ionenstärke bis auf eine Ionenstärke oberhalb von 10⁻² M erhöht wird.

10. Verfahren nach einem der vorhergehenden Ansprüche, **dadurch gekennzeichnet, daß** die LCST des Polymers zwischen 30 und 40°C liegt.

11. Verfahren nach einem der vorhergehenden Ansprüche, **dadurch gekennzeichnet, daß** das erste Monomer (1) ausgewählt ist aus den N-Alkylacrylamiden und den N,N-Dialkylacrylamiden.

12. Verfahren nach Anspruch 11, **dadurch gekennzeichnet, daß** das erste Monomer (1) ausgewählt ist aus N-Isopropylacrylamid, N-Ethylmethacrylamid, N-n-Propylacrylamid, N-n-Propylmethacrylamid, N-Isopropylmethacrylamid, N-Cyclopropylacrylamid, N,N-Diethylacrylamid, N-Methyl-N-isopropylacrylamid, N-Methyl-N-n-propylacrylamid, wobei das erste Monomer vorzugsweise M-Isopropylacrylamid (NIPAM) ist.

13. Verfahren nach einem der vorhergehenden Ansprüche, **dadurch gekennzeichnet, daß** die zweiten funktionellen Monomere (3) ausgewählt sind aus den Acrylderivaten und den Methacrylderivaten, dem Chlorid von 2-Aminoethylmethacrylat (AEM), den Derivaten von N-Vinyl-pyridin, den Derivaten von Trialkylammonium und den Derivaten von Isothiouroniumchlorid.

14. Verfahren nach einem der vorhergehenden Ansprüche, **dadurch gekennzeichnet, daß** das wasserlösliche Vernetzungsreagenz (2) ausgewählt ist aus N,N-Methylenbisacrylamid (MBA) und Ethylenglycoldimethacrylat.

15. Verfahren nach einem der vorhergehenden Ansprüche, **dadurch gekennzeichnet, daß** in Schritt (a) das Polymer durch Radikalenpolymerisation in Gegenwart eines Polymerisationsbeschleunigers durchgeführt wird, der ausgewählt ist aus den neutralen und kationischen, wasserlöslichen Beschleunigern wie beispielsweise das Chlorid von 2,2'-Azobisamidinopropan (V50).

16. Verfahren nach Anspruch 3, **dadurch gekennzeichnet, daß** es nach dem Schritt (d) der Separation einen Desorption genannten Schritt umfaßt, in dem das Nukleinsäurematerial durch Deeorption von dem aus Partikeln bestehenden Träger dissoziiert wird, indem wenigstens einer der Parameter Ionenstärke, pH-Wert und Temperatur wie folgt verändert:
- Erhöhung der Ionenstärke bis auf eine Ionenstärke oberhalb von 10⁻² M,
- Erhöhung des pH-Wertes·bis auf einen pH-Wert oberhalb von 7,
- Erhöhung der Temperatur bis auf eine Temperatur oberhalb des LCST des Polymers.

17. Verfahren nach einem der vorhergehenden Ansprüche, **dadurch gekennzeichnet, daß** der Schritt (d) der Separation mittels einer Technik durchgeführt wird, die ausgewählt ist aus Zentrifugation, Filtration, Präzipitation, Sedimentation und Anlegung eines magnetischen Feldes.
